(19) Europäisches Patentamt
European Patent Office
Office européen des brevets

(11) Veröffentlichungsnummer : **0 494 047 A1**

(12) **EUROPÄISCHE PATENTANMELDUNG**

(21) Anmeldenummer : **91810927.3**

(51) Int. Cl.[5] : **A61K 31/425**

(22) Anmeldetag : **27.11.91**

(30) Priorität : **06.12.90 CH 3868/90**
**20.03.91 CH 844/91**
**03.04.91 CH 986/91**

(43) Veröffentlichungstag der Anmeldung :
**08.07.92 Patentblatt 92/28**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Feige, Ulrich, Dr.**
**Unholzgasse 2**
**CH-4125 Riehen (CH)**
Erfinder : **Wiesenberg, Irmgard, Dr.**
**Hauptstrasse 211**
**W-7858 Weil am Rhein (DE)**
Erfinder : **Widler, Leo, Dr.**
**Melchior Berri-Strasse 11**
**CH-4142 Münchenstein (CH)**
Erfinder : **Ferrini, Pier Giorgio, Dr.**
**Im Rehwechsel 22**
**CH-4102 Binningen (CH)**
Erfinder : **Missbach, Martin, Dr.**
**Cristalinweg 4**
**CH-4310 Rheinfelden (CH)**

(54) **Verwendung von Carbazonen zur Behandlung rheumatoider Erkrankungen.**

(57) Verbindungen der Formel I

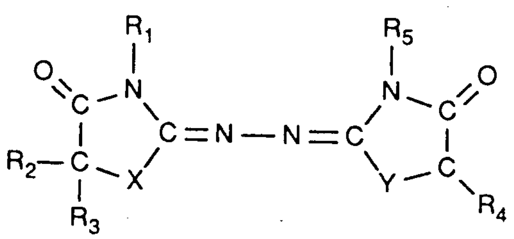

(I),

worin X und Y unabhängig voneinander Thio oder Sulfinyl darstellen, $R_1$ Niederalk-2-en-1-yl, Niederalk-3-en-2-yl, Niederalk-2-in-1-yl oder in 2,3-Stellung durch zu einer Doppelbindung eliminierbare Reste substituiertes Niederalkyl bedeutet, $R_2$ Wasserstoff und $R_3$ unsubstituiertes oder durch einen zusammen mit Wasserstoff $R_2$ zu einer Doppelbindung eliminierbaren Rest substituiertes Methyl bedeutet oder $R_2$ und $R_3$ beide Wasserstoff oder Niederalkyl bedeuten oder gemeinsam Methylen darstellen, $R_4$ für gegebenenfalls geschütztes Hydroxy steht oder Wasserstoff bedeutet und $R_5$ Wasserstoff, Niederalkyl, Niederalk-2-en-1-yl, Niederalk-2-in-1-yl oder in 2,3-Stellung durch zu einer Doppelbindung eliminierbare Reste substituiertes Niederalkyl bedeutet, und ihre pharmazeutisch verwendbaren Salze davon können in neuartiger Weise für Herstellung für die Behandlung von Erkrankungen des rheumatoiden Formenkreises bestimmten pharmazeutischen Präparaten verwendet werden.

EP 0 494 047 A1

Die Erfindung betrifft die Verwendung von Carbazonen der Formel I

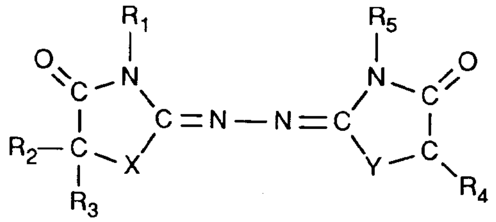

(I),

worin X und Y unabhängig voneinander Thio oder Sulfinyl darstellen, $R_1$ Niederalk-2-en-1-yl, Niederalk-3-en-2-yl, Niederalk-2-in-1-yl oder in 2,3-Stellung durch zu einer Doppelbindung eliminierbare Reste substituiertes Niederalkyl bedeutet, $R_2$ Wasserstoff und $R_3$ unsubstituiertes oder durch einen zusammen mit Wasserstoff $R_2$ zu einer Doppelbindung eliminierbaren Rest substituiertes Methyl bedeutet oder $R_2$ und $R_3$ beide Wasserstoff oder Niederalkyl bedeuten oder gemeinsam Methylen darstellen, $R_4$ für gegebenenfalls geschütztes Hydroxy steht oder Wasserstoff bedeutet und $R_5$ Wasserstoff, Niederalkyl, Niederalk-2-en-1-yl, Niederalk-2-in-1-yl oder in 2,3-Stellung durch zu einer Doppelbindung eliminierbare Reste substituiertes Niederalkyl bedeutet, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

In 2,3-Stellung durch zu einer Doppelbindung eliminierbare Reste substituiertes Niederalkyl ist beispielsweise 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3- (Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl.

Durch einen zusammen mit Wasserstoff $R_2$ zu einer Doppelbindung eliminierbaren Rest substituiertes Methyl ist beispielsweise Diniederalkylaminomethyl oder Halomethyl.

Geschütztes Hydroxy ist beispielsweise Niederalkanoyloxy, Halogenniederalkanoyloxy, Niederalkoxycarbonyloxy, gegebenenfalls durch Niederalkyl, Niederalkoxy, Hydroxy, Halogen und/oder Nitro substituiertes Benzoyloxy, Sulfonyloxy, O-mono- oder O,O-Diniederalkylphosphonyloxy oder Triniederalkylsilyloxy.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise $C_1$-$C_7$-Alkyl, vorzugsweise $C_1$-$C_4$-Alkyl, wie insbesondere Methyl oder in zweiter Linie Äthyl, Propyl, Isopropyl oder Butyl, kann aber auch Isobutyl, Sekundärbutyl, Tertiärbutyl oder eine $C_5$-$C_7$-Alkyl-, wie Pentyl-, Hexyl- oder Heptylgruppe sein.

Niederalkoxy ist beispielsweise $C_1$-$C_7$-Alkoxy, vorzugsweise $C_1$-$C_4$-Alkoxy, wie Methoxy, Äthoxy, Propyloxy, Isopropyloxy oder Butyloxy, kann aber auch Isobutyloxy, Sekundärbutyloxy, Tertiärbutyloxy oder eine Pentyloxy-, Hexyloxy- oder Heptyloxygruppe sein.

Niederalk-2-en-1-yl ist beispielsweise $C_3$-$C_7$-Alk-2-en-1-yl, insbesondere $C_3$-$C_5$-Alk-2-en-1-yl, wie Allyl(Prop-2-en-1-yl) oder Methallyl (2-Methylprop-2-en-1-yl). Niederalk-3-en-2-yl ist beispielsweise $C_3$-$C_7$-Alk-3-en-2-yl, insbesondere $C_3$-$C_5$-Alk-3-en-2-yl, wie But-3-en-2-yl.

Niederalk-2-inyl ist beispielsweise $C_3$-$C_7$-Alk-2-in-1-yl, insbesondere $C_3$-$C_5$-Alk-2-in-1-yl, wie Prop-2-in-1-yl oder 2-Methylprop-2-in-1-yl).

2- oder 3-Aminoniederalkyl ist beispielsweise 2-Amino-$C_3$-$C_7$-alkyl, insbesondere Amino-$C_3$-$C_5$-alkyl, wie 2-Aminopropyl oder 2-Amino-2-methyl-propyl, oder 3-Amino-$C_3$-$C_7$-alkyl, insbesondere 3-Amino-$C_3$-$C_5$-alkyl, wie 3-Aminopropyl oder 3-Amino-2-methyl-propyl.

2- oder 3-Niederalkylaminoniederalkyl ist beispielsweise 2-$C_1$-$C_4$-Alkylamino-$C_3$-$C_7$-alkyl, insbesondere $C_1$-$C_4$-Alkylamino-$C_3$-$C_5$-alkyl, wie 2-$C_1$-$C_4$-Alkylaminopropyl oder 2-$C_1$-$C_4$-Alkylamino-2-methyl-propyl oder 3-$C_1$-$C_4$-Alkylamino-$C_3$-$C_7$-alkyl, insbesondere 3-$C_1$-$C_4$-Alkylamino-$C_3$-$C_5$-alkyl, wie 3-$C_1$-$C_4$-Alkylaminopropyl oder 3-$C_1$-$C_4$-Alkylamino-2-methyl-propyl, wobei $C_1$-$C_4$-Alkyl beispielsweise Methyl, Äthyl, Propyl oder Butyl bedeutet.

Diniederalkylaminomethyl ist beispielsweise N,N-Di-$C_1$-$C_4$-alkylaminomethyl, wie N,N-Dimethylaminomethyl, N,N-Diäthylaminomethyl, N-Äthyl-N-methyl-aminomethyl, N,N-Dipropylaminomethyl, N-Methyl-N-propylaminomethyl, N-Isopropyl-N-methylaminomethyl oder N-Butyl-N-methyl-aminomethyl, kann aber auch N-Isobutyl-N-methyl-aminomethyl, N-Methyl-N-sekundärbutyl-aminomethyl, N-Methyl-N-tertiärbutyl-aminomethyl oder eine N-Methyl-N-pentyl-aminomethyl-, N-Hexyl-N-methylaminomethyl- oder N-Heptyl-N-methyl-aminomethylgruppe sein.

2- oder 3-Diniederalkylaminoniederalkyl ist beispielsweise 2-(N,N-Di-$C_1$-$C_4$-alkylamino)-$C_3$-$C_7$-alkyl, insbesondere 2-(N,N-Di-$C_1$-$C_4$-alkylamino)-$C_3$-$C_5$-alkyl, wie 2-(N,N-Di-$C_1$-$C_4$-alkylamino)propyl oder 2-(N,N-Di-$C_1$-$C_4$-alkylamino)-2-methyl-propyl oder 3-(N,N-Di-$C_1$-$C_4$-alkylamino)-$C_3$-$C_7$-alkyl, insbesondere 3-(N,N-Di-$C_1$-$C_4$-alkylamino)-$C_3$-$C_5$-alkyl, wie 3-(N,N-Di-$C_1$-$C_4$-alkylamino)propyl oder 3-(N,N-Di-$C_1$-$C_4$-alkylamino)-2-methyl-propyl, wobei $C_1$-$C_4$-Alkyl beispielsweise Methyl, Äthyl, Propyl oder Butyl bedeutet.

2- oder 3-Niederalkylenaminoniederalkyl ist beispielsweise 4- bis 7-gliedriges 2-(N,N-Alkylenamino)-$C_3$-$C_7$-alkyl, insbesondere 2-(N,N-Alkylenamino-$C_3$-$C_5$-alkyl, wie 2-(N,N-Alkylenamino)propyl oder 2-(N,N-Alkylenamino)-2-methyl-propyl oder 3-(N,N-Alkylenamino)-$C_3$-$C_7$-alkyl, insbesondere 3-(N,N-Alkylenamino)-$C_3$-$C_5$-alkyl, wie 3-(N,N-Alkylenamino)propyl oder 3-(N,N-Alkylenamino)-2-methyl-propyl, wobei 4-bis 7-gliedriges N,N-Alkylenamino insbesondere Pyrrolidino, Piperidino oder in zweiter Linie Hexahydroazepino oder Octahydroazocino bedeutet.

2- oder 3-(Aza)niederalkylenaminoniederalkyl ist beispielsweise 4- bis 7-gliedriges 2-[N,N-(Aza)alkylenamino]-$C_3$-$C_7$-alkyl, insbesondere 2-[N,N-(Aza)alkylenamino]-$C_3$-$C_5$-alkyl, wie 2-[N,N-(Aza)alkylenamino]propyl oder 2-[N,N-(Aza)alkylenamino]-2-methyl-propyl oder 3-[N,N-(Aza)alkylenamino]-$C_3$-$C_7$-alkyl, insbesondere 3-[N,N-(Aza)alkylenamino]-$C_3$-$C_5$-alkyl, wie 3-[N,N-(Aza)alkylenamino]propyl oder 3-[N,N-(Aza)alkylenamino]-2-methyl-propyl, wobei 4-bis 7-gliedriges N,N-(Aza)alkylenamino insbesondere Piperazino oder N′-$C_1$-$C_4$-Alkyl-, wie N′-Methylpiperazino, oder N′-$C_1$-$C_7$-Alkanoyl-, wie N′-Acetyl- oder N′-Pivaloylpiperazino bedeutet.

2- oder 3-(Oxa)niederalkylenaminoniederalkyl ist beispielsweise 4- bis 7-gliedriges 2-[N,N-(Oxa)alkylenamino]-$C_3$-$C_7$-alkyl, insbesondere 2-[N,N-(Oxa)alkylenamino]-$C_3$-$C_5$-alkyl, wie 2-[N,N-(Oxa)alkylenamino]propyl oder 2-[N,N-(Oxa)alkylenamino]-2-methyl-propyl oder 3-[N,N-(Oxa)alkylenamino]-$C_3$-$C_7$-alkyl, insbesondere3-[N,N-(Oxa)alkylenamino]-$C_3$-$C_5$-alkyl, wie 3-[N,N-(Oxa)alkylenamino]propyl oder 3-[N,N-(Oxa)alkylenamino]-2-methyl-propyl, wobei 4-bis 7-gliedriges N,N-(Oxa)alkylenamino insbesondere Morpholino bedeutet.

2- oder 3-(Thia)niederalkylenaminoniederalkyl ist beispielsweise 4- bis 7-gliedriges, gegebenenfalls S-oxidiertes 2-[N,N-(Thia)alkylenamino]-$C_3$-$C_7$-alkyl, insbesondere 2-[N,N-(Thia)alkylenamino]-$C_3$-$C_5$-alkyl, wie 2-[N,N-(Thia)alkylenamino]propyl oder 2-[N,N-(Thia)alkylenamino]-2-methyl-propyl oder 3-[N,N-(Thia)alkylenamino]-$C_3$-$C_7$-alkyl, insbesondere 3-[N,N-(Thia)alkylenamino]-$C_3$-$C_5$-alkyl, wie 3-[N,N-(Thia)alkylenamino]propyl oder 3-[N,N-(Thia)alkylenamino]-2-methyl-propyl, wobei 4-bis 7-gliedriges, gegebenenfalls S-oxidiertes N,N-(Thia)alkylenamino insbesondere Thiomorpholino oder S-Oxy- bzw. S,S-Dioxythiomorpholino bedeutet.

2- oder 3-Hydroxyniederalkyl ist beispielsweise 2-Hydroxy-$C_3$-$C_7$-alkyl, insbesondere 2-Hydroxy-$C_3$-$C_5$-alkyl, wie 2-Hydroxypropyl oder 2-Hydroxy-2-methyl-propyl, oder 3-Hydroxy-$C_3$-$C_7$-alkyl, insbesondere 3-Hydroxy-$C_3$-$C_5$-alkyl, wie 3-Hydroxypropyl oder 3-Hydroxy-2-methyl-propyl.

Niederalkanoyloxy ist beispielsweise $C_1$-$C_7$-Alkanoyloxy, insbesondere $C_1$-$C_5$-Alkanoyloxy, wie Acetyloxy, Propionyloxy, Butyryloxy, Valeroyloxy oder Pivaloyloxy, kann aber auch $C_6$- oder $C_7$-Alkanoyloxy, wie Caproyloxy sein.

2- oder 3-Niederalkanoyloxyniederalkyl ist beispielsweise 2-($C_1$-$C_7$-Alkanoyloxy)-$C_3$-$C_7$-alkyl, insbesondere 2-($C_1$-$C_7$-Alkanoyloxy)-$C_3$-$C_5$-alkyl, wie 2-($C_1$-$C_7$-Alkanoyloxy)propyl oder 2-($C_1$-$C_7$-Alkanoyloxy)-2-methylpropyl, oder 3-($C_1$-$C_7$-Alkanoyloxy)-$C_3$-$C_7$-alkyl, insbesondere 3-($C_1$-$C_7$-Alkanoyloxy)-$C_3$-$C_5$-alkyl, wie 3-($C_1$-$C_7$-Alkanoyloxy)propyl oder 3-($C_1$-$C_7$-Alkanoyloxy)-2-methyl-propyl, wobei $C_1$-$C_7$-Alkanoyloxy insbesondere $C_1$-$C_4$-Alkanoyloxy, wie Acetyloxy oder Pivaloyloxy, bedeutet.

Niederalkoxycarbonyloxy ist beispielsweise $C_1$-$C_7$-Alkoxycarbonyloxy, vorzugsweise $C_1$-$C_4$-Alkoxycarbonyloxy, wie Methoxycarbonyloxy, Äthoxycarbonyloxy, Propyloxycarbonyloxy, Isopropyloxycarbonyloxy oder Butyloxycarbonyloxy, kann aber auch Isobutyloxycarbonyloxy, Sekundärbutyloxycarbonyloxy, Tertiärbutyloxycarbonyloxy oder eine Pentyloxycarbonyloxy-, Hexyloxycarbonyloxy- oder Heptyloxycarbonyloxygruppe sein.

2- oder 3-Niederalkoxycarbonyloxyniederalkyl ist beispielsweise 2-($C_1$-$C_7$-Alkoxycarbonyloxy)-$C_3$-$C_7$-alkyl, insbesondere 2-($C_1$-$C_7$-Alkoxycarbonyloxy)-$C_3$-$C_5$-alkyl, wie 2-($C_1$-$C_7$-Alkoxycarbonyloxy)propyl oder 2-($C_1$-$C_7$-Alkoxycarbonyloxy)-2-methylpropyl, oder 3-($C_1$-$C_7$-Alkoxycarbonyloxy)-$C_3$-$C_7$-alkyl, insbesondere 3-($C_1$-$C_7$-Alkoxycarbonyloxy)-$C_3$-$C_5$-alkyl, wie 3-($C_1$-$C_7$-Alkoxycarbonyloxy)propyl oder 3-($C_1$-$C_7$-Alkoxycarbonyloxy)-2-methyl-propyl, wobei $C_1$-$C_7$-Alkoxycarbonyloxy insbesondere $C_1$-$C_4$-Alkoxycarbonyloxy, wie Tertiärbutyloxycarbonyl bedeutet.

Triniederalkylsilyloxy ist beispielsweise Tri-$C_1$-$C_7$-alkylsilyloxy, insbesondere Tri-$C_1$-$C_4$-alkylsilyloxy, wie Trimethylsilyloxy oder Tributylsilyloxy, oder $C_4$-$C_7$-Alkyl(di-$C_1$-$C_4$-alkyl)silyloxy, wie 1,2,2-Trimethylpropyl(dimethyl)silyloxy.

2- oder 3-Triniederalkylsilyloxyniederalkyl ist beispielsweise 2-(Tri-$C_1$-$C_7$-alkylsilyloxy)-$C_3$-$C_7$-alkyl, insbesondere 2-(Tri-$C_1$-$C_7$-alkylsilyloxy)-$C_3$-$C_5$-alkyl, wie 2-(Tri-$C_1$-$C_7$-alkylsilyloxy)propyl oder 2-(Tri-$C_1$-$C_7$-alkylsilyloxy)-2-methyl-propyl, oder 3-(Tri-$C_1$-$C_7$-alkylsilyloxy)-$C_3$-$C_7$-alkyl, insbesondere 3-(Tri-$C_1$-$C_7$-alkylsilyloxy)-$C_3$-$C_5$-alkyl, wie 3-(Tri-$C_1$-$C_7$-alkylsilyloxy)propyl oder 3-(Tri-$C_1$-$C_7$-alkylsilyloxy)-2-methyl-propyl, wobei Tri-$C_1$-$C_7$-alkylsilyloxy insbesondere Tri-$C_1$-$C_4$-alkylsilyloxy, wie Trimethylsilyloxy oder Tributylsilyloxy, oder $C_4$-$C_7$-Al-

kyl(di-$C_1$-$C_4$-alkyl)silyloxy, wie 1,2,2-Trimethylpropyl(dimethyl)silyloxy bedeutet.

Halogen ist beispielsweise Halogen der Atomnummer bis und mit 35, wie Chlor oder Fluor, ferner Brom.

2- oder 3-Haloniederalkyl ist beispielsweise 2-Halo-$C_3$-$C_7$-alkyl, insbesondere 2-Halo-$C_3$-$C_5$-alkyl, wie 2-Halopropyl oder 2-Halo-2-methyl-propyl oder 3-Halo-$C_3$-$C_7$-alkyl, insbesondere 3-Halo-$C_3$-$C_5$-alkyl, wie 3-Halopropyl oder 3-Halo-2-methyl-propyl, wobei Halo beispielsweise Halogen der Atomnummer bis und mit 35, wie Chlor oder Fluor, ferner Brom bedeutet.

Halomethyl ist beispielsweise Halomethyl, worin Halo die der Atomnummer bis und mit 35 aufweist und beispielsweise Chlor oder Fluor, ferner Brom bedeutet.

Halogenniederalkanoyloxy ist beispielsweise Chloracetyloxy.

O-Mono- oder O,O-Diniederalkylphosphonyloxy ist beispielsweise O-Mono-$C_1$-$C_4$-alkylphosphonyloxy, wie O-Methyl- oder O-Äthylphosphonyloxy.

Die Verbindungen der Formel I sind mit Ausnahme derjenigen, in denen $R_4$ sauren Charakter aufweist und die deshalb amphoter sind, basisch. Basische Verbindungen der Formel I können Säureadditionssalze, die erwähnten amphoteren Verbindungen der Formel I ferner Salze mit Basen bilden.

Pharmazeutisch verwendbare Saüreadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate.

Pharmazeutisch verwendbare Salze von Verbindungen der Formel I mit Basen sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Äthyl- oder Diäthylamin, Mono-, Di- oder Tri-(hydroxyniederalkyl)aminen, wie Äthanol-, Diäthanoloder Triäthanolamin, Tris-(hydroxymethyl)methylamin oder 2-Hydroxytertiärbutylamin, oder N-(Hydroxyniederalkyl)-N,N-diniederalkyl- aminen bzw. N-(Polyhydroxyniederalkyl)-N-niederalkylaminen, wie 2-(Dimethylamino)äthanol oder D-Glucamin, oder quaternären aliphatischen Ammoniumhydroxiden, z.B. Tetrabutylammoniumhydroxid.

Die Verbindungen der Formel I und auf an sich bekannten Methoden beruhende Verfahren zu Ihrer Herstellung sind bekannt und beispielsweise in GB-1,325,061, US-4,697,020, DE-2,632,746, DE-2,632,747, EP-085,275 und EP-22,515 beschrieben. Neue Verbindungen der Formel I und Ihre pharmazeutisch verwendbaren Salze können in analoger Weise hergestellt werden wie dort beschrieben.

Die Verbindungen der Formel I und ihre pharmazeutisch verwendbaren Salze weisen gemäss den genannten Druckschriften tumorhemmende Eigenschaften auf und wurden als tumorhemmende Arzneimittelwirkstoffe, insbesondere zur Behandlung von neoplastischen Erkrankungen bei Warmblütern vorgeschlagen.

Die vorliegende Erfindung beruht auf der überraschenden Feststellung, dass die Verbindungen der Formel I und ihre pharmazeutisch verwendbaren Salze zusätzlich zu ihrer bekannten tumorhemmenden Wirkung, die meist im täglichen Dosisbereich von etwa 10 bis etwa 250 mg/kg i.p. auftritt, bereits bei sehr geringer, noch nicht tumorhemmender Dosierung, ausgeprägte antiarthritische Eigenschaften aufweisen. Diese können *in vivo* beispielsweise am Modell der Adjuvans-Arthritis der Ratte nach I. Wiesenberg et al. Clin. Exp. Immunol. 78, 245 (1989) im Dosisbereich ab etwa 0,1 bis etwa 0,3 mg/kg p.o. oder i.p. nachgewiesen werden.

Der Mechanismus der antiarthritischen Wirkung der Verbindungen der Formel I und ihrer pharmazeutisch verwendbaren Salze ist derzeit noch nicht genau bekannt. Es kann jedoch ausgeschlossen werden, dass ein direkter immunsuppressiver Mechanismus via Lymphozytotoxizität oder Myelosuppression vorliegt, weil selbst Dosierungen, die mehr als 100-mal höher sind als die antiarthritisch wirksame Dosis, zu keiner sonst zu erwartenden massiven Suppression der lymphatischen Organe (Thymus, Milz) oder zu einer Leukopenie führen. Bei der Therapie rheumatoider Erkrankungen mit immunsuppressiv wirkenden Zytostatika, z.B. mit Cyclophosphamid, sind Lymphozytotoxizität und Myelosuppression bekannte unerwünschte Nebenwirkungen.

Hingegen konnte nachgewiesen werden, dass die Verbindungen der Formel I und ihre pharmazeutisch verwendbaren Salze in humanen Monozyten die Synthese des Zytokins Interleukin-1 hemmen. Interleukin-1 ist ein Entzündungsmediator, der in akuten und chronischen Entzündungsprozessen eine Schlüsselrolle spielt. Es ist deshalb davon auszugehen, dass die Verbindungen der Formel I und ihre pharmazeutisch verwendbaren Salze in Dosierungen, die niedriger sind als die zytostatisch wirksame Dosis, Eigenschaften besitzen, die die antiarthritischen Effekte bewirken. Ein ursächlicher Zusammenhang mit der genannten Zytokinsynthesehemmung könnte bestehen, obwohl auch andere, noch nicht bekannte immunmodulatorische Mechanismen nicht ausgeschlossen werden können.

Die Verbindungen der Formel I und ihre pharmazeutisch verwendbaren Salze können deshalb zur Behandlung von Erkrankungen des rheumatoiden Formenkreises im Sinne des "preliminary proposal of the Glossary Committee of the American Rheumatism Association"eingesetzt werden. Zu diesen gehören insbesondere die rheumatoide Arthritis, die juvenile Arthritis, die ankylosierende Spondylitis und andere seronegative Spondylathritiden, z.B. Spondylarthritis bei Colitis ulcerosa und Morbus Crohn, aber auch reaktive Arthritiden, Kollagenkrankheiten wie Lupus erythematosus, degenerative rheumatische Erkrankungen, extraartikuläre rheumatische und pararheumatische Erkrankungen, z.B. Gicht und Osteoporose.

Die Erfindung betrifft in erster Linie die Verwendung von Verbindungen der Formel I, worin X und Y unabhängig voneinander Thio oder Sulfinyl darstellen, $R_1$ Niederalk-2-en-1-yl, Niederalk-3-en-2-yl, Niederalk-2-in-1-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl bedeutet, $R_2$ Wasserstoff und $R_3$ Wasserstoff, Niederalkyl, Diniederalkylaminoniederalkyl oder Halomethyl bedeutet oder $R_2$ und $R_3$ gemeinsam Methylen darstellen, $R_4$ Hydroxy, Niederalkanoyloxy, Halogenniederalkanoyloxy, Niederalkoxycarbonyloxy, gegebenenfalls durch Niederalkyl, Niederalkoxy, Hydroxy, Halogen und/oder Nitro substituiertes Benzoyloxy, Sulfonyloxy, O-mono- oder O,O-Diniederalkylphosphonyloxy, Triniederalkylsilyloxy oder Wasserstoff bedeutet und $R_5$ Wasserstoff, Niederalkyl, Niederalk-2-en-1-yl, Niederalk-2-in-1-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Silyloxyniederalkyl oder 2- oder 3-Haloniederalkyl bedeutet, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft in erster Linie beispielsweise die Verwendung von Verbindungen der Formel I, worin X und Y Thio darstellen, $R_1$ Niederalk-2-en-1-yl, Niederalk-3-en-2-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl bedeutet, $R_2$ Wasserstoff und $R_3$ Wasserstoff, Niederalkyl, Diniederalkylaminoniederalkyl oder Halomethyl bedeutet oder $R_2$ und $R_3$ gemeinsam Methylen darstellen, $R_4$ Hydroxy, Niederalkanoyloxy, Halogenniederalkanoyloxy, Niederalkoxycarbonyloxy, gegebenenfalls durch Niederalkyl, Niederalkoxy, Hydroxy, Halogen und/oder Nitro substituiertes Benzoyloxy, Sulfonyloxy, O-mono- oder O,O-Diniederalkylphosphonyloxy, Triniederalkylsilyloxy oder Wasserstoff bedeutet und $R_5$ Wasserstoff, Niederalkyl, Niederalk-2-en-1-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Silyloxyniederalkyl oder 2- oder 3-Haloniederalkyl bedeutet, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft in vor allem die Verwendung von Verbindungen der Formel I, worin die Reste X und Y unabhängig voneinander Thio oder Sulfinyl darstellen, $R_1$ $C_3$-$C_7$-Alk-2-en-1-yl, wie Allyl oder Methallyl, $C_3$-$C_7$-Alk-3-en-2-yl, wie But-3-en-2-yl, $C_3$-$C_7$-Alk-2-in-1-yl, insbesondere $C_3$-$C_5$-Alk-2-in-1-yl, wie Prop-2-in-1-yl oder 2-Methylprop-2-in-1-yl, oder 2- oder 3-Halo-$C_3$-$C_7$-alkyl, wie 2-Bromisobutyl, bedeutet, $R_2$ Wasserstoff bedeutet und $R_3$ $C_1$-$C_4$-Alkyl, wie Methyl, Di-$C_1$-$C_4$-alkylaminomethyl, wie Dimethylaminomethyl, oder Halomethyl, wie 3-Bromisobutyl, oder $R_2$ und $R_3$ Wasserstoff oder $C_1$-$C_4$-Alkyl, wie Methyl, bedeuten oder gemeinsam für Methylen stehen, $R_4$ Hydroxy, $C_1$-$C_7$-Alkanoyloxy, wie Acetyloxy oder Pivaloyloxy, Sulfonyloxy, O-$C_1$-$C_4$-Alkyl- oder O,O-Di-$C_1$-$C_4$-alkylphosphonyloxy, wie O-Methyl- oder O,O-Dimethylphosphonyloxy, Tri-$C_1$-$C_7$-alkylsilyloxy, wie Trimethyl- oder Triisobutylsilyloxy, oder gegebenenfalls durch $C_1$-$C_4$-Alkyl, wie Methyl, $C_1$-$C_4$-Alkoxy, wie Methoxy, Hydroxy und/oder Halogen substituiertes Benzoyloxy bedeutet und $R_5$ Wasserstoff, $C_1$-$C_4$-Alkyl, wie Methyl, $C_3$-$C_7$-Alk-2-en-1-yl, wie Allyl oder Methallyl, $C_3$-$C_7$-Alk-2-in-1-yl, insbesondere $C_3$-$C_5$-Alk-2-in-1-yl, wie Prop-2-in-1-yl oder 2-Methylprop-2-in-1-yl, oder 2- oder 3-Halo-$C_3$-$C_7$-alkyl, wie 3-Bromisobutyl, darstellt, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft vorzugsweise die Verwendung von Verbindungen der Formel I, worin X und Y unabhängig voneinander Thio oder Sulfinyl darstellen, $R_1$ $C_3$-$C_7$-Alk-2-en-1-yl, wie Allyl oder Methallyl, $C_3$-$C_7$-Alk-3-en-2-yl, wie But-3-en-2-yl, $C_3$-$C_7$-Alk-2-in-1-yl, wie Prop-2-in-1-yl oder 2-Methylprop-2-in-1-yl, oder 2-Halo-$C_3$-$C_7$-alkyl, wie 2-Bromisobutyl, bedeutet, $R_2$ Wasserstoff und $R_3$ Di-$C_1$-$C_4$-alkylaminomethyl, wie Dimethylaminomethyl, bedeutet oder $R_2$ und $R_3$ beide für Methyl oder gemeinsam für Methylen stehen, $R_4$ Hydroxy, $C_1$-$C_7$-Alkanoyloxy, wie Acetyloxy oder Pivaloyloxy, Sulfonyloxy, O-$C_1$-$C_4$-Alkylphosphonyloxy, wie O-Methylphosphonyloxy, Tri-$C_1$-$C_4$-alkylsilyloxy, wie Trimethylsilyloxy oder Tributylsilyloxy, oder $C_4$-$C_7$-Alkyl(di-$C_1$-$C_4$-alkyl)silyloxy, wie 1,2,2-Trimethylpropyl(dimethyl)silyloxy darstellt und $R_5$ $C_1$-$C_4$-Alkyl, wie Methyl, $C_3$-$C_7$-Alk-2-in-1-yl, wie Prop-2-in-1-yl oder 2-Methylprop-2-in-1-yl, oder $C_3$-$C_7$-Alk-2-en-1-yl, wie Allyl oder Methallyl, bedeutet, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft vorzugsweise beispielsweise die Verwendung von Verbindungen der Formel I, worin X und Y Thio darstellen, $R_1$ $C_3$-$C_7$-Alk-2-en-1-yl, wie Allyl oder Methallyl, $C_3$-$C_7$-Alk-3-en-2-yl, wie But-3-en-2-yl, oder 2-Halo-$C_3$-$C_7$-alkyl, wie 2-Bromisobutyl, bedeutet, $R_2$ Wasserstoff und $R_3$ Di-$C_1$-$C_4$-alkylaminomethyl, wie Dimethylaminomethyl, bedeutet oder $R_2$ und $R_3$ beide für Methyl oder gemeinsam für Methylen stehen, $R_4$ Hydroxy, $C_1$-$C_7$-Alkanoyloxy, wie Acetyloxy oder Pivaloyloxy, Sulfonyloxy, O-$C_1$-$C_4$-Alkylphosphonyloxy, wie O-Methylphosphonyloxy, Tri-$C_1$-$C_4$-alkylsilyloxy, wie Trimethylsilyloxy oder Tributylsilyloxy, oder $C_4$-$C_7$-Alkyl(di-$C_1$-$C_4$-alkyl)silyloxy, wie 1,2,2-Trimethylpropyl(dimethyl)silyloxy darstellt und $R_5$ $C_1$-$C_4$-Alkyl, wie Methyl, oder $C_3$-$C_7$-Alk-2-en-1-yl, wie Allyl oder Methallyl, bedeutet, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft insbesondere die Verwendung von Verbindungen der Formel I, worin X und Y unabhängig voneinander Thio oder Sulfinyl darstellen, $R_1$ $C_3$-$C_7$-Alk-2-en-1-yl, wie Allyl oder Methallyl, $C_3$-$C_7$-Alk-2-in-1-yl, wie Prop-2-in-1-yl oder 2-Methylprop-2-in-1-yl, oder 2-Halo-$C_3$-$C_7$-alkyl, wie 2-Bromisobutyl, bedeutet, $R_2$ Wasserstoff oder Methyl bedeutet, $R_3$ für Methyl steht, $R_4$ Hydroxy, $C_1$-$C_7$-Alkanoyloxy, wie Acetyloxy oder Pivaloyloxy, Sulfonyloxy oder Tri-$C_1$-$C_4$-alkylsilyloxy, wie Trimethylsilyloxy oder Tributylsilyloxy, oder $C_4$-$C_7$-Alkyl(di-$C_1$-$C_4$-alkyl)silyloxy, wie 1,2,2-Trimethylpropyl(dimethyl)silyloxy, darstellt und $R_5$ $C_1$-$C_4$-Alkyl, wie Methyl, bedeutet, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft in allererster Linie die Verwendung von Verbindungen der Formel I, worin X und Y unabhängig voneinander Thio oder Sulfinyl darstellen, $R_1$ $C_3$-$C_5$-Alk-2-en-1-yl, wie Allyl oder Methallyl, $C_3$-$C_5$-Alk-2-in-1-yl, wie Prop-2-in-1-yl oder 2-Methylprop-2-in-1-yl, oder 2-Halo-$C_3$-$C_5$-alkyl, wie 2-Bromisobutyl, bedeutet, $R_2$ Wasserstoff und $R_3$ Di-$C_1$-$C_4$-alkylaminomethyl, wie Dimethylaminomethyl, bedeutet oder $R_2$ und $R_3$ gemeinsam für Methylen stehen, $R_4$ Wasserstoff bedeutet und $R_5$ $C_1$-$C_4$-Alkyl, wie Methyl, darstellt, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft in allererster Linie beispielsweise die Verwendung von Verbindungen der Formel I, worin X und Y Thio bedeuten, $R_1$ $C_3$-$C_7$-Alk-2-en-1-yl, wie Allyl oder Methallyl, oder 2-Halo-$C_3$-$C_7$-alkyl, wie 2-Bromisobutyl, bedeutet, $R_2$ Wasserstoff und $R_3$ Di-$C_1$-$C_4$-alkylaminomethyl, wie Dimethylaminomethyl, bedeutet oder $R_2$ und $R_3$ gemeinsam für Methylen stehen, $R_4$ Wasserstoff bedeutet und $R_5$ $C_1$-$C_4$-Alkyl, wie Methyl, darstellt, und ihrer pharmazeutisch verwendbaren Salze zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Erfindung betrifft namentlich die Verwendung von

3-Methallyl-2-(3-methyl-4-oxo-thiazolidin-2-ylidenhydrazono)-5-methylen-thiazolidin-4-on,

3-Allyl-2-(3-methyl-4-oxo-thiazolidin-2-ylidenhydrazono)-5-methylen-thiazolidin-4-on,

3-Methallyl-2-(3-methyl-4-oxo-thiazolidin-2-ylidenhydrazono)-5-dimethylaminomethylthiazolidin-4-on und seines Hydrochlorides,

3-Methallyl-2-(3-methyl-5-hydroxy-4-oxo-thiazolidin-2-ylidenhydrazono)-5-methylthiazolidin-4-on,

2-(5-Acetoxy-3-methyl-4-oxo-thiazolidin-2-ylidenhydrazono)-3-methallyl-5-methylthiazolidin-4-on,

3-Methallyl-2-(3-methyl-5-pivaloyloxy-4-oxo-thiazolidin-2-ylidenhydrazono)-5-methylthiazolidin-4-on,
3-Methallyl-2-(3-methyl-5-sulfoxy-4-oxo-thiazolidin-2-ylidenhydrazono)-5-methylthiazolidin-4-on und seines Natriumsalzes,
Methyl-[3-methyl-2-(3-methallyl-5-methyl-4-oxo-thiazolidin-2-ylidenhydrazono)-4-oxothiazolidin-5-yl]phosphat, insbesondere Methyl-[3-methyl-2-(3-methallyl-5(R*)-methyl-4-oxo-thiazolidin-2-ylidenhydrazono)-4-oxo-thiazolidin-5(R*)-yl]phosphat,und seines Natrium-, Kalium-, Ammonium-, Triäthanolammonium-, 2-Hydroxyäthylammonium- oder Tertiärbutylammoniumsalzes,
3-Methallyl-2-(3-methyl-5-trimethylsilyloxy-4-oxo-thiazolidin-2-ylidenhydrazono)-5,5-dimethyl-thiazolidin-4-on,
3-Methallyl-2-(3-methyl-5-tributylsilyloxy-4-oxo-thiazolidin-2-ylidenhydrazono)-5-methyl-thiazolidin-4-on,
Methyl-[3-methyl-2-(3-allyl-4-oxo-thiazolidin-2-ylidenhydrazono)-4-oxo-thiazolidin-5-yl]phosphat und seines Natrium- oder Tertiärbutylammoniumsalzes,
Methyl-[3-methyl-2-(3-allyl-5-methyl-4-oxo-thiazolidin-2-ylidenhydrazono)-4-oxothiazolidin-5-yl]phosphat und seines Natriumsalzes,
3-Methallyl-2-{3-methyl-5-[tertiäbutyl(dimethyl)silyloxy]-4-oxo-thiazolidin-2-ylidenhydrazono}-5-methyl-thiazolidin-4-on,
Methyl-[3-methyl-2-(3-allyl-5,5-dimethyl-4-oxo-thiazolidin-2-ylidenhydrazono)-4-oxothiazolidin-5-yl]phosphat seines Natriumsalzes,
3-Methallyl-2-{3-methyl-5-[tertiärbutyl(dimethyl)silyloxy]-4-oxo-thiazolidin-2-ylidenhydrazono}-5,5-dimethyl-thiazolidin-4-on,
3-Methallyl-2-(3-methyl-5-triisopropylsilyloxy-4-oxo-thiazolidin-2-ylidenhydrazono)-5-methyl-thiazolidin-4-on,
3-Methallyl-2-{3-methyl-5-[(2,3-dimethylbut-2-yl)(dimethyl)silyloxy]-4-oxo-thiazolidin-2-ylidenhydrazono}-5-methyl-thiazolidin-4-on,
5-Hydroxymethyl-3-methallyl-2-(3-methyl-4-oxo-thiazolidin-2-ylidenhydrazono)-thiazolidin-4-on,
Phosphorsäure-dimethylamid-[3-methyl-2-(3-methallyl-5-methyl-4-oxo-thiazolidin-2-ylidenhydrazono)-4-oxo-thiazolidin-5-yl]ester,
[3-Methyl-2-(3-methallyl-5-methyl-4-oxo-thiazolidin-2-ylidenhydrazono)-5-thiazolidin-5-yloxy]-2-oxo-1,3,2-oxazaphospholidin,
3-Methallyl-2-(4-oxo-thiazolidin-2-ylidenhydrazono)-thiazolidin-4-on,
3-Methallyl-2-(1,4-dioxothiazolidin-2-ylidenhydrazono)-1-oxy-thiazolidin-4-on
3-Allyl-2-(3-methyl-5-hydroxy-4-oxo-thiazolidin-2-ylidenhydrazono)-thiazolidin-4-on,
3-Methallyl-2-(3-allyl-5-hydroxy-4-oxo-thiazolidin-2-ylidenhydrazono)-5,5-dimethylthiazolidin-4-on,
3-(But-3-en-2-yl)-2-(4-oxo-thiazolidin-2-ylidenhydrazono)-thiazolidin-4-on,
3-Methallyl-2-(3-methyl-4-oxo-thiazolidin-2-ylidenhydrazono)-5-methyl-thiazolidin-4-on,
3-Allyl-2-(3-methyl-4-oxo-thiazolidin-2-ylidenhydrazono)-thiazolidin-4-on,
3-(But-3-en-2-yl)-2-(3-methyl-4-oxo-thiazolidin-2-ylidenhydrazono)-thiazolidin-4-on,
3-Methallyl-2-(3-methyl-4-oxo-thiazolidin-2-ylidenhydrazono)-thiazolidin-4-on,
3-Methallyl-2-(4-oxo-thiazolidin-2-ylidenhydrazono)-thiazolidin-4-on,
3-Methallyl-2-(3-allyl-4-oxo-thiazolidin-2-ylidenhydrazono)-5,5-dimethyl-1-oxo-thiazolidin-4-on,
3-Allyl-2-(3-methyl-4-oxo-thiazolidin-2-ylidenhydrazono)-1-oxo-thiazolidin-4-on,
3-Methallyl-2-(3-allyl-1,4-dioxo-thiazolidin-2-ylidenhydrazono)-5,5-dimethyl-1-oxo-thiazolidin-4-on,
3-Allyl-2-(3-methyl-1,4-dioxo-thiazolidin-2-ylidenhydrazono)-thiazolidin-4-on,
Dimethyl-[3-methyl-2-(3-methallyl-5-methyl-4-oxo-thiazolidin-2-ylidenhydrazono)-4-oxo-thiazolidin-5-yl]phosphat und
3-(Prop-2-inyl-2-(3-methyl-4-oxo-thiazolidin-2-ylidenhydrazono)-thiazolidin-4-on zur Herstellung für die Behandlung von Erkrankungen des rheumatoiden Formenkreises bestimmter pharmazeutischer Präparate, enthaltend eine Verbindung der Formel I oder ein pharmazeutisch verwendbares Salz davon neben üblichen pharmazeutischen Hilfsstoffen.

Die Verbindungen der Formel I und ihre pharmazeutisch verwendbaren Salze werden zur Herstellung von pharmazeutischen Präparaten verwendet, welche die erfindungsgemässe Verbindung oder pharmazeutisch verwendbare Salze davon enthalten. Dabei handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, femer Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsaüre, Talk, Stearinsäure oder Salze davon, wie Magnesium- oder Calciumstearat, und/oder Polyäthylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyäthylenglykol, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyäthylenglykol oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykol oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 5 mg bis etwa 1000 mg, insbesondere von etwa 10 mg bis etwa 200 mg zu veranschlagen. Diese kann auf auf einmal gegeben oder auf mehrere, z.B. 2 bis 4 Einzeldosen verteilt werden.

Pharmazeutische Präparate in Dosiseinheitsform enthalten somit von etwa 5 mg bis etwa 250 mg, insbesondere von etwa 10 mg bis etwa 50 mg.

Die nachfolgen Beispiele dienen zur Illustration der Erfindung, sollen diese jedoch in ihrem Geltungsbereich in keiner Weise einschränken

Beispiel 1:

Tabletten, enthaltend je 10 mg 3-Allyl-2-(3-methyl-4-oxo-thiazolidin-2-ylidenhydrazono)-thiazolidin-4-on oder ein Salz davon, können wie folgt hergestellt werden:

Zusammensetzung (10000 Tabletten)

| | |
|---|---|
| Wirkstoff | 100,0 g |
| Lactose | 450,0 g |
| Kartoffelstärke | 350,0 g |
| Gelatine | 10,0 g |
| Talk | 60,0 g |

| | |
|---|---|
| Magnesiumstearat | 10,0 g |
| Siliciumdioxid (hochdispers) | 20,0 g |
| Äthanol | q. s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 100,0 mg Gewicht und 50,0 mg Wirkstoffgehalt, die gewünschtenfalls mit Teilkerben zur feineren Anpassung der Dosierung versehen sein können.

Beispiel 2:

Gelatinesteckkapseln, enthaltend 20 mg Wirkstoff, z.B. 3-Allyl-2-(3-methyl-4-oxo-thiazolidin-2-ylidenhydrazono)-thiazolidin-4-on oder ein Salz davon, können z.B. folgendermassen hergestellt werden:

Zusammensetzung (für 1000 Kapseln)

| | |
|---|---|
| Wirkstoff | 20,0 g |
| Lactose | 240,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 300 mg der erhaltenen Formulierung in Gelatinesteckkapseln der Grösse 0 abgefüllt.

Beispiel 3:

Gelatinesteckkapseln, enthaltend 100 mg Wirkstoff, z.B. 3-Allyl-2-(3-methyl-4-oxo-thiazolidin-2-ylidenhydrazono)-thiazolidin-4-on oder ein Salz davon, können z.B. folgendermassen hergestellt werden:

Zusammensetzung (für 1000 Kapseln)

| | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

Beispiel 4:

Lacktabletten, enthaltend je 50 mg 3-Allyl-2-(3-methyl-4-oxo-thiazolidin-2-ylidenhydrazono)-thiazolidin-4-on oder ein Salz davon, können wie folgt hergestellt werden:

Zusammensetzung (für 1000 Lacktabletten)

| | |
|---|---|
| Wirkstoff | 50,0 g |

| | |
|---|---|
| Lactose | 100,0 g |
| Maisstärke | 70,0 g |
| Talk | 10,0 g |
| Calciumstearat | 2,0 g |
| Hydroxypropylmethylcellulose | 2,36 g |
| Schellack | 0,64 g |
| Wasser | q.s. |
| Methylenchlorid | q. s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 240 mg) verpresst und diese mit einer lösung der Hydroxypropyl-methylcellulose und des Schellacks in Methylenchlorid lackiert;
Endgewicht der Lacktablette: 283 mg.

Beispiel 5:

Eine 0,2%-ige Injektions- oder Infusionslösung von 3-Allyl-2-(3-methyl-4-oxo-thiazolidin-2-ylidenhydrazono)-thiazolidin-4-on oder eines Salzes davon, kann beispielsweise folgendermassen hergestellt werden:

Zusammensetzunp (für 1000 Ampullen)

| | |
|---|---|
| Wirkstoff | 5,0 g |
| Natriumchlorid | 22,5 g |
| Phosphatpuffer pH= 7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

Beispiel 6:

1 %-ige Salbe (O/W-Emulsion), als Wirkstoff z.B 3-Allyl-2-(3-methyl-4-oxo-thiazolidin-2-ylidenhydrazono)-thiazolidin-4-on oder ein Salz davon

Zusammensetzung:

| | |
|---|---|
| Wirkstoff | 1,0 g |
| Cetylalkohol | 3,0 g |
| Glycerin | 6,0 g |
| Methylparaben | 0, 18 g |
| Propylparaben | 0,05 g |
| Arlacel 60 | 0,6 g |
| Tween 60 | 4,4 g |
| Stearinsäure | 9,0 g |
| Isopropylpalmitat | 2,0 g |
| Paraffinöl dickflüssig | 10,0 g |
| Wasser entmin. q.s. ad | 100,0 g |

Beispiel 7:

1%-iges Gel, als Wirkstoff z.B.
3-Allyl-2-(3-methyl-4-oxo-thiazolidin-2-ylidenhydrazono)-thiazolidin-4-on oder e ein Salz davon, enthaltend

Zusammensetzung:

| | |
|---|---|
| Wirkstoff | 1,0 g |
| Carbopol 934 P | 1,0 g |
| Glycerin | 3,0 g |
| Isopropanol | 25,0 g |
| Softigen 767 | 0,2 g |
| Wasser entmin. q.s. ad | 100,0 g |

Beispiel 8:

In analoger Weise wie in den vorstehenden Beispielen 1 bis 7 beschrieben kann man auch pharmazeutische Präparate, enthaltend
3-Methallyl-2-(3-methyl-4-oxo-thiazolidin-2-ylidenhydrazono)-5-methylen-thiazolidin-4-on,
3-Methally-2-(3-methyl-4-oxo-thiazolidin-2-ylidenhydrazono)-5-dimethylaminomethyl-thiazolidin-4-on oder sein Hydrochlorid,
3-Methallyl-2-(3-methyl-5-hydroxy-4-oxo-thiazolidin-2-ylidenhydrazono)-5-methyl-thiazolidin-4-on,
2-(5-Acetoxy-3-methyl-4-oxo-thiazolidin-2-ylidenhydrazono)-3-methallyl-5-methyl-thiazolidin-4-on,
3-Methallyl-2-(3-methyl-5-pivaloyloxy-4-oxo-thiazolidin-2-ylidenhydrazono)-5-methyl-thiazolidin-4-on,
3-Methallyl-2-(3-methyl-5-sulfoxy-4-oxo-thiazolidin-2-ylidenhydrazono)-5-methyl-thiazolidin-4-on oder sein Natriumsalz,
Methyl-[3-methyl-2-(3-methallyl-5-methyl-4-oxo-thiazolidin-2-ylidenhydrazono)-4-oxo-thiazolidin-5-yl]phosphat, insbesondere Methyl-[3-methyl-2-(3-methallyl-5(R*)-methyl-4-oxo-thiazolidin-2-ylidenhydrazono)-4-oxo-thiazolidin-5(R*)-yl]phosphat,oder sein Natrium-, Kalium-, Ammonium-, Triäthanolammonium-, 2-Hydroxyäthylammoniumoder Tertiärbutylammoniumsalz,
3-Methallyl-2-(3-methyl-5-trimethylsilyloxy-4-oxo-thiazolidin-2-ylidenhydrazono)-5,5-dimethyl-thiazolidin-4-on,
3-Methallyl-2-(3-methyl-5-tributylsilyloxy-4-oxo-thiazolidin-2-ylidenhydrazono)-5-methyl-thiazolidin-4-on,
Methyl-[3-methyl-2-(3-allyl -4-oxo-thiazolidin-2-ylidenhydrazono)-4-oxo-thiazolidin-5-yl]phosphat oder sein Natrium- oder Tertiärbutylammoniumsalz,
Methyl-[3-methyl-2-(3-allyl-5-methyl-4-oxo-thiazolidin-2-ylidenhydrazono)-4-oxo-thiazolidin-5-yl]phosphat oder sein Natriumsalz,
3-Methallyl-2-{3-methyl-5-[tertiärbutyl(dimethyl)silyloxy]-4-oxo-thiazolidin-2-ylidenhydrazono}-5-methyl-thiazolidin-4-on,
Methyl-[3-methyl-2-(3-allyl-5,5-dimethyl-4-oxo-thiazolidin-2-ylidenhydrazono)-4-oxo-thiazolidin-5-yl]phosphat oder sein Natriumsalz,
3-Methallyl-2-{3-methyl-5-[tertiärbutyl(dimethyl)silyloxy]-4-oxo-thiazolidin-2-ylidenhydrazono}-5,5-dimethyl-thiazolidin-4-on,
3-Methallyl-2-(3-methyl-5-triisopropylsilyloxy-4-oxo-thiazolidin-2-ylidenhydrazono)-5-methyl-thiazolidin-4-on,
3-Methallyl-2-{3-methyl-5-[(2,3-dimethylbut-2-yl)(dimethyl)silyloxy]-4-oxo-thiazolidin-2-ylidenhydrazono}-5-methyl-thiazolidin-4-on,
5-Hydroxymethyl-3-methallyl-2-(3-methyl-4-oxo-thiazolidin-2-ylidenhydrazono)-thiazolidin-4-on,
Phosphorsäure-dimethylamid-[3-methyl-2-(3-methallyl-5-methyl-4-oxo-thiazolidin-2-ylidenhydrazono)-4-oxo-thiazolidin-5-yl]ester,
[3-Methyl-2-(3-methallyl-5-methyl-4-oxo-thiazolidin-2-ylidenhydrazono)-5-thiazolidin-5-yloxy]-2-oxo-1,3,2-oxazaphospholidin,
3-Methallyl-2-(4-oxo-thiazolidin-2-ylidenhydrazono)-thiazolidin-4-on,
3-Allyl-2-(3-methyl-5-hydroxy-4-oxo-thiazolidin-2-ylidenhydrazono)-thiazolidin-4-on,
3-Methallyl-2-(3-allyl-5-hydroxy-4-oxo-thiazolidin-2-ylidenhydrazono)-5,5-dimethyl-thiazolidin-4-on,
3-(But-3-en-2-yl)-2-(4-oxo-thiazolidin-2-ylidenhydrazono)-thiazolidin-4-on,
3-Methallyl-2-(3-methyl-4-oxo-thiazolidin-2-ylidenhydrazono)-5-methyl-thiazolidin-4-on,
3-Allyl-2-(3-methyl-4-oxo-thiazolidin-2-ylidenhydrazono)-thiazolidin-4-on,
3-(But-3-en-2-yl)-2-(3-methyl-4-oxo-thiazolidin-2-ylidenhydrazono)-thiazolidin-4-on,
3-Methallyl-2-(3-methyl-4-oxo-thiazolidin-2-ylidenhydrazono)-thiazolidin-4-on,
3-Methallyl-2-(4-oxo-thiazolidin-2-ylidenhydrazono)-thiazolidin-4-on,
3-Methallyl-2-(1,4-dioxothiazolidin-2-ylidenhydrazono)-1-oxy-thiazolidin-4-on
3-Methallyl-2-(3-allyl-4-oxo-thiazolidin-2-ylidenhydrazono)-5,5-dimethyl-1-oxo-thiazolidin-4-on,
3-Allyl-2-(3-methyl-4-oxo-thiazolidin-2-ylidenhydrazono)-1-oxo-thiazolidin-4-on,

3-Methallyl-2-(3-allyl-1,4-dioxo-thiazolidin-2-ylidenhydra zono)-5,5-dimethyl-1-oxo-thiazolidin-4-on,
3-Allyl-2-(3-methyl-1,4-dioxo-thiazolidin-2-ylidenhydrazono)-thiazolidin-4-on,
Dimethyl-[3-methyl-2-(3-methallyl-5-methyl-4-oxo-thiazolidin-2-ylidenhydrazono)-4-oxo-thiazolidin-5-yl]phosphat oder
3-(Prop-2-inyl)-2-(3-methyl-4-oxo-thiazolidin-2-ylidenhydrazono)-thiazolidin-4-on herstellen.

## Patentansprüche

1. Verwendung von Verbindungen Formel I

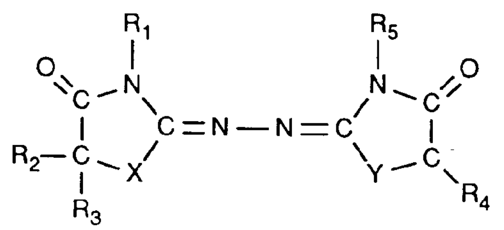

(I),

worin X und Y unabhängig voneinander Thio oder Sulfinyl darstellen, $R_1$ Niederalk-2-en-1-yl, Niederalk-3-en-2-yl, Niederalk-2-in-1-yl oder in 2,3-Stellung durch zu einer Doppelbindung eliminierbare Reste substituiertes Niederalkyl bedeutet, $R_2$ Wasserstoff und $R_3$ unsubstituiertes oder durch einen zusammen mit Wasserstoff $R_2$ zu einer Doppelbindung eliminierbaren Rest substituiertes Methyl bedeutet oder $R_2$ und $R_3$ beide Wasserstoff oder Niederalkyl bedeuten oder gemeinsam Methylen darstellen, $R_4$ für gegebenenfalls geschütztes Hydroxy steht oder Wasserstoff bedeutet und $R_5$ Wasserstoff, Niederalkyl, Niederalk-2-en-1-yl, Niederalk-2-in-1-yl oder in 2,3-Stellung durch zu einer Doppelbindung eliminierbare Reste substituiertes Niederalkyl bedeutet, und ihrer pharmazeutisch verwendbaren Salze zur Herstellung von für die Behandlung von Erkrankungen des rheumatoiden Formenkreisesbestimmten pharmazeutischen Präparaten.

2. Verwendung gemäss Anspruch 1 zur Herstellung pharmazeutischer Präparate zur enteralen bzw. parenteralen Verabreichung.

3. Verwendung gemäss Anspruch 1 zur Herstellung pharmazeutischer Präparate in Dosiseinheitsform, wie Dragées, Tabletten, Kapseln oder Suppositorien, ferner Injektions- oder Infusionslösungen.

4. Verwendung gemäss Anspruch 1 zur Herstellung pharmazeutischer Präparate gemäss einem der Ansprüche 1 bis 3, enthalten von etwa 5 mg bis etwa 250 mg einer Verbindung der Formel I oder eines pharmazeutisch verwendbaren Salzes davon.

5. Verwendung gemäss Anspruch 1 zur Herstellung pharmazeutischer Präparate gemäss einem der Ansprüche 1 bis 3, enthalten von etwa 10 mg bis etwa 50 mg einer Verbindung der Formel I oder eines pharmazeutisch verwendbaren Salzes davon.

6. Verwendung gemäss einem der Ansprüche 1 bis 5, dadurch gekennzeichnet, dass man eine Verbindung der Formel I, worin X und Y unabhängig voneinander Thio oder Sulfinyl darstellen, $R_1$ Niederalk-2-en-1-yl, Niederalk-3-en-2-yl, Niederalk-2-in-1-yl, 2- oder 3-Amino-, 2- oder 3-Niederalkylamino- oder 2- oder 3-Diniederalkylaminoniederalkyl, 2-oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl bedeutet, $R_2$ Wasserstoff und $R_3$ Wasserstoff, Niederalkyl, Diniederalkylaminoniederalkyl oder Halomethyl bedeutet oder $R_2$ und $R_3$ gemeinsam Methylen darstellen, $R_4$ Hydroxy, Niederalkanoyloxy, Halogenniederalkanoyloxy, Niederalkoxycarbonyloxy, gegebenenfalls durch Niederalkyl, Niederalkoxy, Hydroxy, Halogen und/oder Nitro substituiertes Benzoyloxy, Sulfonyloxy, O-mono- oder O,O-Diniederalkylphosphonyloxy, Triniederalkylsilyloxy oder Wasserstoff bedeutet und $R_5$

Wasserstoff, Niederalkyl, Niederalk2-en-1-yl, Niederalk-2-in-1-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Silyloxyniederalkyl oder 2- oder 3-Haloniederalkyl bedeutet, oder ein pharmazeutisch verwendbares Salz davon wählt.

7. Verwendung gemäss einem der Ansprüche 1 bis 5, dadurch gekennzeichnet, dass man eine Verbindung der Formel I, worin X und Y Thio darstellen, $R_1$ Niederalk-2-en-1-yl, Niederalk-3-en-2-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Triniederalkylsilyloxyniederalkyl oder 2- oder 3-Haloniederalkyl bedeutet, $R_2$ Wasserstoff und $R_3$ Wasserstoff, Niederalkyl, Diniederalkylaminoniederalkyl oder Halomethyl bedeutet oder $R_2$ und $R_3$ gemeinsam Methylen darstellen, $R_4$ Hydroxy, Niederalkanoyloxy, Halogenniederalkanoyloxy, Niederalkoxycarbonyloxy, gegebenenfalls durch Niederalkyl, Niederalkoxy, Hydroxy, Halogen und/oder Nitro substituiertes Benzoyloxy, Sulfonyloxy, O-mono- oder O,O-Diniederalkylphosphonyloxy, Triniederalkylsilyloxy oder Wasserstoff bedeutet und $R_5$ Wasserstoff, Niederalkyl, Niederalk-2-en-1-yl, 2- oder 3-Amino-, 2- oder 3-Niederalkylamino- oder 2- oder 3-Diniederalkylaminoniederalkyl, 2- oder 3-Niederalkylenamino-, 2- oder 3-(Aza)niederalkylenamino-, 2- oder 3-(Oxa)niederalkylenamino- oder 2- oder 3-(Thia)niederalkylenaminoniederalkyl, 2- oder 3-Hydroxy-, 2- oder 3-Niederalkanoyloxy-, 2- oder 3-Niederalkoxycarbonyloxy- oder 2- oder 3-Silyloxyniederalkyl oder 2- oder 3-Haloniederalkyl bedeutet, oder ein pharmazeutisch verwendbares Salz davon wählt.

8. Verwendung gemäss einem der Ansprüche 1 bis 5, dadurch gekennzeichnet, dass man eine Verbindung der Formel I, worin X und Y unabhängig voneinander Thio oder Sulfinyl darstellen, $R_1$ $C_3$-$C_7$-Alk-2-en-1-yl, $C_3$-$C_7$-Alk-3-en-2-yl, $C_3$-$C_7$-Alk-2-in-1-yl oder 2- oder 3-Halo-$C_3$-$C_7$-alkyl bedeutet, $R_2$ Wasserstoff bedeutet und $R_3$ $C_1$-$C_4$-Alkyl, Di-$C_1$-$C_4$-alkylaminomethyl oder Halomethyl oder $R_2$ und $R_3$ Wasserstoff oder $C_1$-$C_4$-Alkyl bedeuten oder gemeinsam für Methylen stehen, $R_4$ Hydroxy, $C_1$-$C_7$-Alkanoyloxy, Sulfonyloxy, O-$C_1$-$C_4$-Alkyl- oder O,O-Di-$C_1$-$C_4$-alkylphosphonyloxy, Tri-$C_1$-$C_7$-alkylsilyloxy oder gegebenenfalls durch $C_1$-$C_4$-Alkyl, $C_1$-$C_4$-Alkoxy, Hydroxy und/oder Halogen substituiertes Benzoyloxy bedeutet und $R_5$ Wasserstoff, $C_1$-$C_4$-Alkyl, $C_3$-$C_7$-Alk-2-en-1-yl, $C_3$-$C_7$-Alk-2-in-1-yl oder 2- oder 3-Halo-$C_3$-$C_7$-alkyl darstellt, oder ein pharmazeutisch verwendbares Salz davon wählt.

9. Verwendung gemäss einem der Ansprüche 1 bis 5, dadurch gekennzeichnet, dass man eine Verbindung der Formel I, worin X und Y unabhängig voneinander Thio oder Sulfinyl darstellen, $R_1$ $C_3$-$C_7$-Alk-2-en-1-yl, $C_3$-$C_7$-Alk-3-en-2-yl, $C_3$-$C_7$-Alk-2-in-1-yl oder 2-Halo-$C_3$-$C_7$-alkyl bedeutet, $R_2$ Wasserstoff und $R_3$ Di-$C_1$-$C_4$-alkylaminomethyl bedeutet oder $R_2$ und $R_3$ beide für Methyl oder gemeinsam für Methylen stehen, $R_4$ Hydroxy, $C_1$-$C_7$-Alkanoyloxy, Sulfonyloxy, O-$C_1$-$C_4$-Alkylphosphonyloxy, Tri-$C_1$-$C_4$-alkylsilyloxy oder $C_4$-$C_7$-Alkyl(di-$C_1$-$C_4$-alkyl)silyloxy darstellt und $R_5$ $C_1$-$C_4$-Alkyl, $C_3$-$C_7$-Alk-2-in-1-yl, oder $C_3$-$C_7$-Alk-2-en-1-yl bedeutet, oder ein pharmazeutisch verwendbares Salz davon wählt.

10. Verwendung gemäss einem der Ansprüche 1 bis 5, dadurch gekennzeichnet, dass man eine Verbindung der Formel I, worin X und Y Thio darstellen, $R_1$ $C_3$-$C_7$-Alk-2-en-1-yl, $C_3$-$C_7$-Alk-3-en-2-yl, oder 2-Halo-$C_3$-$C_7$-alkyl bedeutet, $R_2$ Wasserstoff und $R_3$ Di-$C_1$-$C_4$-alkylaminomethyl bedeutet oder $R_2$ und $R_3$ beide für Methyl oder gemeinsam für Methylen stehen, $R_4$ Hydroxy, $C_1$-$C_7$-Alkanoyloxy, Sulfonyloxy, O-$C_1$-$C_4$-Alkylphosphonyloxy, Tri-$C_1$-$C_4$-alkylsilyloxy oder $C_4$-$C_7$-Alkyl(di-$C_1$-$C_4$-alkyl)silyloxy darstellt und $R_5$ $C_1$-$C_4$-Alkyl oder $C_3$-$C_7$-Alk-2-en-1-yl bedeutet, oder ein pharmazeutisch verwendbares Salz davon wählt.

11. Verwendung gemäss einem der Ansprüche 1 bis 5, dadurch gekennzeichnet, dass man eine Verbindung der Formel I, worin X und Y unabhängig voneinander Thio oder Sulfinyl darstellen, $R_1$ $C_3$-$C_7$-Alk-2-en-1-yl, $C_3$-$C_7$-Alk-2-in-1-yl oder 2-Halo-$C_3$-$C_7$-alkyl bedeutet, $R_2$ Wasserstoff oder Methyl bedeutet, $R_3$ für Methyl steht, $R_4$ Hydroxy, $C_1$-$C_7$-Alkanoyloxy, Sulfonyloxy, Tri-$C_1$-$C_4$-alkylsilyloxy, oder $C_4$-$C_7$-Alkyl(di-$C_1$-$C_4$-alkyl)silyloxy darstellt und $R_5$ $C_1$-$C_4$-Alkyl bedeutet, oder ein pharmazeutisch verwendbares Salz davon wählt.

12. Verwendung gemäss einem der Ansprüche 1 bis 5, dadurch gekennzeichnet, dass man eine Verbindung der Formel I, worin X und Y unabhängig voneinander Thio oder Sulfinyl darstellen, $R_1$ $C_3$-$C_5$-Alk-2-en-1-yl,

$C_3$-$C_5$-Alk-2-in-1-yl oder 2-Halo-$C_3$-$C_5$-alkyl bedeutet, $R_2$ Wasserstoff und $R_3$ Di-$C_1$-$C_4$-alkylaminomethyl bedeutet oder $R_2$ und $R_3$ gemeinsam für Methylen stehen, $R_4$ Wasserstoff bedeutet und $R_5$ $C_1$-$C_4$-Alkyl darstellt, oder ein pharmazeutisch verwendbares Salz davon wählt.

**13.** Verwendung gemäss einem der Ansprüche 1 bis 5, dadurch gekennzeichnet, dass man eine Verbindung der Formel I, worin $R_1$ $C_3$-$C_7$-Alk-2-en-1-yl oder 2-Halo-$C_3$-$C_4$-alkyl bedeutet, $R_2$ Wasserstoff und $R_3$ Di-$C_1$-$C_4$-alkylaminomethyl bedeutet oder $R_2$ und $R_3$ gemeinsam für Methylen stehen, $R_4$ Wasserstoff bedeutet und $R_5$ $C_1$-$C_4$-Alkyl darstellt, oder ein pharmazeutisch verwendbares Salz davon wählt.

**14.** Verwendung gemäss einem der Ansprüche 1 bis 5, dadurch gekennzeichnet, dass man als Verbindung der Formel I
3-Methallyl-2-(3-methyl-4-oxo-thiazolidin-2-ylidenhydrazono)-5-methylen-thiazolidin-4-on,
3-Allyl-2-(3-methyl-4-oxo-thiazolidin-2-ylidenhydrazono)-thiazolidin-4-on,
3-Allyl-2-(3-methyl-4-oxo-thiazolidin-2-ylidenhydrazono)-5-methylen-thiazolidin-4-on,
3-Methallyl-2-(3-methyl-4-oxo-thiazolidin-2-ylidenhydrazono)-5-dimethylaminonmethyl-thiazolidin-4-on,
3-Methallyl-2-(3-methyl-5-hydroxy-4-oxo-thiazolidin-2-ylidenhydrazono)-5-methyl-thiazolidin-4-on,
2-(5-Acetoxy-3-methyl-4-oxo-thiazolidin-2-ylidenhydrazono)-3-methallyl-5-methyl-thiazolidin-4-on,
3-Methallyl-2-(3-methyl-5-pivaloyloxy-4-oxo-thiazolidin-2-ylidenhydrazono)-5-methyl-thiazolidin-4-on,
3-Methallyl-2-(3-methyl-5-sulfoxy-4-oxo-thiazolidin-2-ylidenhydrazono)-5-methyl-thiazolidin-4-on,
3-Methallyl-2-(3-methyl-5-trimethylsilyloxy-4-oxo-thiazolidin-2-ylidenhydrazono)-5,5-dimethyl-thiazolidin-4-on,
3-Methallyl-2-(3-methyl-5-tributylsilyloxy-4-oxo-thiazolidin-2-ylidenhydrazono)-5-methyl-thiazolidin-4-on,
Methyl-[3-methyl-2-(3-allyl-4-oxo-thiazolidin-2-ylidenhydrazono)-4-oxo-thiazolidin-5-yl]phosphat,
Methyl-[3-methyl-2-(3-allyl-5-methyl-4-oxo-thiazolidin-2-ylidenhydrazono)-4-oxo-thiazolidin-5-yl]phosphat,
3-Methallyl-2-{3-methyl-5-[tertiärbutyl(dimethyl)silyloxy]-4-oxo-thiazolidin-2-ylidenhydrazono}-5-methyl-thiazolidin-4-on,
Methyl-[3-methyl-2-(3-allyl-5,5-dimethyl-4-oxo-thiazolidin-2-ylidenhydrazono)-4-oxo-thiazolidin-5-yl]phosphat,
3-Methallyl-2-{3-methyl-5-[tertiärbutyl(dimethyl)silyloxy]-4-oxo-thiazolidin-2-ylidenhydrazono}-5,5-dimethyl-thiazolidin-4-on,
3-Methallyl-2-(3-methyl-5-triisopropylsilyloxy-4-oxo-thiazolidin-2-ylidenhydrazono)-5-methyl-thiazolidin-4-on,
3-Methallyl-2-{3-methyl-5-[(2,3-dimethylbut-2-yl)(dimethyl)silyloxy]-4-oxo-thiazolidin-2-ylidenhydrazono}-5-methyl-thiazolidin-4-on oder jeweils ein pharmazeutisch verwendbares Salz davon wählt.

**15.** Verwendung gemäss einem der Ansprüche 1 bis 5, dadurch gekennzeichnet, dass man als Verbindung der Formel I
Methyl-[3-methyl-2-(3-methallyl-5(R*)-methyl-4-oxo-thiazolidin-2-ylidenhydrazono)-4-oxo-thiazolidin-5(R*)-yl]phosphat,
5-Hydroxymethyl-3-methallyl-2-(3-methyl-4-oxo-thiazolidin-2-ylidenhydrazono)-thiazolidin-4-on,
Phosphorsäure-dimethylamid-[3-methyl-2-(3-methallyl-5-methyl-4-oxo-thiazolidin-2-ylidenhydrazono)-4-oxo-thiazolidin-5-yl]ester,
[3-Methyl-2-(3-methallyl-5-methyl-4-oxo-thiazolidin-2-ylidenhydrazono)-5-thiazolidin-5-yloxy]-2-oxo-1,3,2-oxazaphospholidin,
3-Methallyl-2-(4-oxo-thiazolidin-2-ylidenhydrazono)-thiazolidin-4-on,
3-Allyl-2-(3-methyl-5-hydroxy-4-oxo-thiazolidin-2-ylidenhydrazono)-thiazolidin-4-on,
3-Methallyl-2-(3-allyl-5-hydroxy-4-oxo-thiazolidin-2-ylidenhydrazono)-5,5-dimethyl-thiazolidin-4-on,
3-(But-3-en-2-yl)-2-(4-oxo-thiazolidin-2-ylidenhydrazono)-thiazolidin-4-on,
3-Methallyl-2-(3-methyl-4-oxo-thiazolidin-2-ylidenhydrazono)-5-methyl-thiazolidin-4-on,
3-(But-3-en-2-yl)-2-(3-methyl-4-oxo-thiazolidin-2-ylidenhydrazono)-thiazolidin-4-on,
3-Methallyl-2-(3-methyl-4-oxo-thiazolidin-2-ylidenhydrazono)-thiazolidin-4-on oder
3-Methallyl-2-(4-oxo-thiazolidin-2-ylidenhydrazono)-thiazolidin-4-on
oder jeweils ein pharmazeutisch verwendbares Salz davon wählt.

**16.** Verwendung gemäss einem der Ansprüche 1 bis 5, dadurch gekennzeichnet, dass man als Verbindung der Formel I bzw. als S-Monoxid davon
3-Methallyl-2-(3-allyl-4-oxo-thiazolidin-2-ylidenhydrazono)-5,5-dimethyl-1-oxo-thiazolidin-4-on,
3-Allyl-2-(3-methyl-4-oxo-thiazolidin-2-ylidenhydrazono)-1-oxo-thiazolidin-4-on,
3-Allyl-2-(3-methyl-1,4-dioxo-thiazolidin-2-ylidenhydrazono)-thiazolidin-4-on,
Dimethyl-[3-methyl-2-(3-methallyl-5-methyl-4-oxo-thiazolidin-2-ylidenhydrazono)-4-oxo-thiazolidin-5-yl]phosphat oder
3-(Prop-2-inyl-2-(3-methyl-4-oxo-thiazolidin-2-ylidenhydrazono)-thiazolidin-4-on
oder jeweils ein pharmazeutisch verwendbares Salz davon wählt.

**17.** Verwendung gemäss einem der Ansprüche 1 bis 5, dadurch gekennzeichnet, dass man als Verbindung der Formel I Methyl-[3-methyl-2-(3-methallyl-5-methyl-4-oxo-thiazolidin-2-ylidenhydrazono)-4-oxo-thiazolidin-5-yl] phosphat oder ein pharmazeutisch verwendbares Salz davon wählt.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 91 81 0927

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| Y | EUROPEAN JOURNAL OF CANCER, Band 15, Nr. 5, May 1979, Seiten 755-762, GB; V.C. JORDAN et al.: "The mode of action of the antitumour agent GP 48,989 in the rat" * Zusammenfassung; Seiten 758-759 * --- | 1-17 | A 61 K 31/425 |
| Y | PROC. INT. CONGR. CHEMOTHER., Band 16, 1983, Seiten 257/66,257/70; K. SCHIEWECK et al.: "CGP 19 984 A: Antitumour activity in vivo and in vitro" * Zusammenfassung; Seiten 257/69 * --- | 1-17 | |
| Y | "The Merck Manual of Diagnosis and Therapy", 15. Ausgabe, 1987, Seiten 1239-1246, Merck & Co., Inc., Rahway, NJ, US * Seite 1245 * --- -/- | 1-17 | |
| | | | RECHERCHIERTE SACHGEBIETE (Int. Cl.5) |
| | | | A 61 K |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche: 1-17

Nicht recherchierte Patentansprüche:

Grund für die Beschränkung der Recherche:

Die Gegenstände der Ansprüche 1-17 sind nicht durch pharmakologische Daten gestützt. Durch das Fehlen pharmakologischer Daten wird die Beurteilung des technischen Gegenstandes der Ansprüche und des Standes der Technik fragwürdig und subjektiv. Es kann deshalb durchaus sein, dass der nächstliegende Stand der Technik nicht im Recherchenbericht zitiert wurde. (Art. 84) EPÜ).

| Recherchenort | Abschlußdatum der Recherche | Prüfer |
|---|---|---|
| DEN HAAG | 30-03-1992 | KRAUTBAUER B. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0409)

Seite 2

Europäisches Patentamt

# EUROPÄISCHER TEILRECHERCHENBERICHT

Nummer der Anmeldung
EP 91 81 0927

| EINSCHLÄGIGE DOKUMENTE | | | KLASSIFIKATION DER ANMELDUNG (Int. Cl.5) |
|---|---|---|---|
| Kategorie | Kennzeichnung des Dokuments mit Angabe, soweit erforderlich der maßgeblichen Teile | Betrifft Anspruch | |
| Y | "The Merck Manual of Diagnosis and Therapy", 15. Ausgabe, 1987, Seiten 1221-1228, Merck & Co., Inc., Rahway, NJ, US<br>* Seiten 1221-1222 *<br>--- | 1-17 | |
| D,Y | US-A-4 697 020 (A. STORNI et al.)<br>* Zusammenfassung; Spalte 5; Patentansprüche 1-4 *<br>--- | 1-17 | |
| D,Y | EP-A-0 085 275 (CIBA-GEIGY AG)<br>* Zusammenfassung; Seiten 3,28; Patentansprüche 1-13 *<br>--- | 1-17 | RECHERCHIERTE SACHGEBIETE (Int. Cl.5) |
| D,Y | GB-A-1 325 061 (CIBA-GEIGY AG)<br>* Seite 1; Patentansprüche 1-8 *<br>----- | 1-17 | |

EPO FORM 1503 03.82 (P0412)